# EUROPÄISCHE PATENTANMELDUNG

(11) **EP 3 822 018 A1**
(43) Veröffentlichungstag der Anmeldung: **19.05.2021**
(21) Anmeldenummer: 19209532.1
(22) Anmeldetag: 15.11.2019
(51) Int. Cl.: B23K 20/10, B29C 65/08, B06B 1/10, A61B 17/32, B06B 3/00, B26D 7/08

(54) **ULTRASCHALLKOMPONENTE, VORRICHTUNG ZUM BEARBEITEN VON WERKSTÜCKEN UND VERFAHREN ZUM BEARBEITEN VON WERKSTÜCKEN**

(71) Anmelder: Telsonic Holding AG, 9552 Bronschhofen (CH)
(72) Erfinder: Solenthaler, Peter, 9543 St. Margarethen (CH)
(74) Vertreter: Hepp Wenger Ryffel AG

(57) **Zusammenfassung**

Eine Sonotrode (1) zum Bearbeiten von Werkstücken (W) mit Ultraschallschwingungen weist einen Sonotrodenkörper (10) auf. An einer Schalleinleitungsseite (11) mit einer ersten Stirnfläche (12) werden longitudinale Schwingungen (SL) in den Sonotrodenkörper (10) eingeleitet. Zwischen der Schalleinleitungsseite (11) und einer dieser gegenüberliegenden Bearbeitungsseite (13) ist eine Konversionsstruktur (15) angeordnet, mit der aus den longitudinalen Schwingungen (SL) Querschwingungen (ST1) mit einer Schwingungskomponente in einer Ebene (E) senkrecht zur Längsachse (L) erzeugt werden. Zwischen der Konversionsstruktur (15) und der Bearbeitungsseite (13) ist eine Dämpfungsstruktur (18) vorgesehen, welche die Longitudinalschwingungen (SL) reduziert.

## Beschreibung

Die Erfindung betrifft eine Ultraschallkomponente, insbesondere eine Sonotrode oder einen Booster, eine Vorrichtung und ein Verfahren zum Bearbeiten von Werkstücken mit den Merkmalen der Oberbegriffe der unabhängigen Patentansprüche.

Es ist bekannt, Werkstücke durch Einleiten von Ultraschallschwingungen zu bearbeiten. Typische Anwendungen sind das Verbinden von Werkstücken durch Schweissen, das Ausschneiden von Werkstücken oder das Behandeln von Pulvern, wie beispielsweise beim Sieben.

Ultraschallschwingungen werden durch einen Konverter erzeugt, der in einer Längsrichtung schwingt. Zum Verbinden von Werkstücken, insbesondere von Werkstücken aus Metall oder Kunststoff, ist es bekannt, Ultraschallschwingungen in einer Richtung parallel zur Oberfläche der Werkstücke in diese einzuleiten.

Aus WO 95/23668 sind ein Verfahren und eine Vorrichtung zum Verschweissen von Metallteilen bekannt, bei denen ein Sonotrodenkörper in Drehschwingungen versetzt wird. Ein Nachteil dieser Anordnung besteht darin, dass trotz der erzeugten Drehschwingung immer noch longitudinale Komponenten vorhanden sind, welche zu einer Beschädigung des Werkstücks oder zu einer unerwünschten Dämpfung führen können.

Die Erzeugung von torsionalen Schwingungen durch Einleiten von longitudinalen Schwingungen ist ausserdem auch aus US 4,663,556, US 5,662,766, EP 1 103 238, US 2006/004396 oder US 2011/278988 bekannt.

In WO 2012/069413 A1 wurde daher vorgeschlagen, eine Sonotrode so auszubilden und anzuregen, dass die gesamte Sonotrode zu einer Torsionsschwingung mit vernachlässigbar kleinem Längsschwingungsanteil anregbar ist. Zu diesem Zweck werden Schwingungen tangential zum Sonotrodenkörper eingeleitet. Mit dieser Lösung lassen sich longitudinale Komponenten vermeiden und gute Schweissresultate erzielen. Die Konstruktion ist allerdings verhältnismässig aufwendig.

Es ist daher die Aufgabe der vorliegenden Erfindung, die Nachteile des Bekannten zu vermeiden, insbesondere eine Ultraschallkomponente, insbesondere eine Sonotrode, eine Vorrichtung und ein Verfahren zum Bearbeiten von Werkstücken zu schaffen, bei denen eine Schwingung in eine Richtung parallel zur Oberfläche des Werkstücks mit möglichst geringem longitudinalem Anteil erzeugbar ist, welche einfach und kostengünstig herstellbar sind und welche im Betrieb zuverlässig sind.

Erfindungsgemäss werden diese Aufgaben mit einer Ultraschallkomponente, einer Vorrichtung und einem Verfahren mit den Merkmalen der kennzeichnenden Teile der unabhängigen Ansprüche gelöst.

Die erfindungsgemässe Ultraschallkomponente dient zum Bearbeiten von Werkstücken mit Ultraschallschwingungen. Die Ultraschallkomponente ist typischerweise eine Sonotrode oder ein Booster. Die Ultraschallkomponente weist einen Sonotrodenkörper mit einer Längsachse auf. Der Sonotrodenkörper weist eine Schalleinleitungsseite und eine Bearbeitungsseite auf. Der Einfachheit halber wird nachstehend der Begriff Sonotrodenkörper auch in Zusammenhang mit anderen Ultraschallkomponenten, z. B. einem Booster, verwendet. Die Schalleinleitungsseite ist mit einer ersten Stirnfläche versehen. Die Bearbeitungsseite ist mit einer zweiten Stirnfläche versehen.

Auf der Schalleinleitungsseite sind longitudinale Schwingungen in einer Richtung parallel zur Längsachse in den Sonotrodenkörper einleitbar.

Zwischen der Schalleinleitungsseite und der Bearbeitungsseite ist eine Konversionsstruktur angeordnet. Mit der Konversionsstruktur sind aus den eingeleiteten longitudinalen Schwingungen Querschwingungen mit einer Schwingungskomponente in einer Ebene senkrecht zur Längsachse erzeugbar.

Erfindungsgemäss ist zwischen der Konversionsstruktur und der Bearbeitungsseite eine Dämpfungsstruktur vorgesehen. Die Dämpfungsstruktur ist so ausgebildet, dass damit die longitudinalen Schwingungen auf der Bearbeitungsseite reduziert werden.

In der Konversionsstruktur werden auf an sich bekannte Art und Weise die eingeleiteten longitudinalen Schwingungen zu Querschwingungen konvertiert (vgl. beispielsweise WO 95/23668). Mit der Dämpfungsstruktur wird sichergestellt, dass longitudinale Schwingungen nicht oder höchstens in einem vernachlässigbaren Umfang auf die Bearbeitungsseite übertragen werden. Die Dämpfungsstruktur ist so ausgebildet, dass in Querrichtung gesehen eine möglichst geringe Dämpfung vorliegt, sodass die Querschwingungen möglichst ungedämpft auf die Bearbeitungsseite übertragen werden.

Gemäss einer bevorzugten Ausführungsform ist der Sonotrodenkörper als Hohlkörper ausgebildet. Es ist aber auch denkbar, den Sonotrodenkörper teilweise oder ganz als Vollkörper auszubilden. Insbesondere kann auch je nach geplanter Anwendung ein Hohlkörper oder ein voller Körper bevorzugt sein.

Bevorzugt ist der Sonotrodenkörper rotationssymmetrisch ausgebildet, insbesondere mit einem kreisförmigen Querschnitt. In diesem Fall sind die Querschwingungen insbesondere torsionale Schwingungen. Alternativ dazu kann der Sonotrodenkörper auch nicht rotationssymmetrisch ausgebildet sein und beispielsweise einen ellipsenförmigen Querschnitt aufweisen.

Es ist aber auch denkbar, den Sonotrodenkörper in Form eines rechteckigen Blocks auszubilden. In diesem Fall sind die Querschwingungen transversale Schwingungen. Solche rechteckigen Sonotrodenkörper sind insbesondere als Schneidmesser oder längliche Schweisssonotroden, beispielsweise zum Verbinden und/oder Ausschneiden von Stücken aus Folienbahnen, verwendbar.

Torsionssonotroden sind beispielsweise zum Verschweissen von Kunststoffteilen oder Metallteilen einsetzbar. Typische Anwendungen können das Einschweissen von Sensorhaltern aus Kunststoff in Stossstangen von Automobilen oder das Verschweissen von Litzen untereinander oder von Litzen auf Anschlussteile sein. Grundsätzlich ist die Anwendung der erfindungsgemässen Ultraschallkomponente aber nicht auf bestimmte Einsatzgebiete beschränkt.

Wenn der Sonotrodenkörper rotationssymmetrisch und insbesondere kreisförmig ausgebildet ist, weist er typischerweise einen Durchmesser von weniger als einem Viertel der longitudinalen Wellenlänge auf, d. h. bei einer Frequenz von 20 kHz typischerweise weniger als 60 mm, bevorzugt weniger als 50 mm und besonders bevorzugt etwa 25 mm bis 35 mm auf. Es hat sich gezeigt, dass bei solchen, verhältnismässig geringen Durchmessern besonders stabile Schwingungsverhalten erzielbar sind. Typischerweise werden Ultraschallschwingungen mit einer Frequenz von 15 kHz bis 50 kHz, bevorzugt von 25 kHz bis 35kHz eingeleitet.

Die Konversionsstruktur ist bevorzugt in Form von Materialaussparungen an einer Aussenfläche des Sonotrodenkörpers ausgebildet. Die Materialaussparungen können sich entlang einer Schraubenlinie erstrecken. Insbesondere können die Materialaussparungen in Form von Konversionsschlitzen ausgebildet sein. Die Anzahl der Konversionsschlitze kann im Bereich von 3 bis 12 liegen und beträgt bevorzugt 6. Es ist aber auch denkbar, Materialaussparungen in Form von einzelnen Löchern auf einer Schraubenlinie vorzusehen.

Die Schraubenlinie kann mit einer konstanten Steigung oder mit einer sich veränderlichen Steigung angeordnet sein. Im Fall einer sich veränderlichen Steigung sind die Materialaussparungen entlang einer Kurve mit einer Krümmung auf der Aussenfläche des Sonotrodenkörpers angeordnet. Die Schraubenlinie hat bevorzugt einen Winkel von etwa 45° bezogen auf die Längsachse des Sonotrodenkörpers.

Alternativ zu Materialaussparungen ist es auch denkbar, die Konversionsstruktur in Form von Materialanhäufungen an der Aussenfläche und/oder an einer Innenfläche des Sonotrodenkörpers auszubilden. Insbesondere können solche Materialanhäufungen bei Sonotrodenkörpern vorgesehen werden, welche durch additive Fertigungsverfahren hergestellt sind.

Die Materialaussparungen können sich im Fall eines Hohlkörpers durch die ganze Wand des Sonotrodenkörpers hindurch erstrecken. Es ist aber auch denkbar, die Materialaussparungen nur als Vertiefungen auf der Aussenfläche eines Sonotrodenkörpers vorzusehen, insbesondere im Zusammenhang mit einem nicht als Hohlkörper ausgebildeten Sonotrodenkörper. Es sind auch Konversionsstrukturen mit einer Kombination von Materialaussparungen und Materialanhäufungen oder Kombinationen von Materialaussparungen unterschiedlicher Form von wie vorstehend beschrieben denkbar.

Die Dämpfungsstruktur ist typischerweise durch eine Materialschwächung im Sonotrodenkörper gebildet. Dabei kann es sich bevorzugt um eine Materialschwächung in Form von Dämpfungsschlitzen handeln. Die Dämpfungsschlitze erstrecken sich insbesondere in einer Richtung senkrecht zur Längsachse des Sonotrodenkörpers. Aufgrund der Dämpfungsschlitze ist die Schalleinleitungsseite mit der Bearbeitungsseite nur noch über zwischen den Dämpfungsschlitzen angeordnete Stege verbunden. Diese Stege sind stabil und übertragen die Querschwingungen. Gleichzeitig ist aufgrund des reduzierten oder fehlenden Materials im Bereich der Dämpfungsschlitze die Übertragung von longitudinalen Schwingungen von der Schalleinleitungsseite auf die Bearbeitungsseite weitgehend unterbunden. Die Dämpfungsstruktur hat eine Filterfunktion für die longitudinalen Schwingungen, sodass nur Schwingungen mit Querkomponenten übertragen werden. Es ist auch denkbar, mehrere Reihen von Dämpfungsschlitzen vorzusehen. Die Anzahl der Dämpfungsschlitze kann im Bereich von 3 bis 12 liegen und beträgt bevorzugt 6.

Selbstverständlich sind aber auch andere Arten von Dämpfungen denkbar. Anstelle von Dämpfungsschlitzen können andere Arten von Materialschwächungen vorgesehen sein, beispielsweise durch Materialbearbeitung, welche das Elastizitätsmodul hinsichtlich Verformungen in Längsrichtung reduziert. Es ist auch denkbar, die Dämpfungsstruktur durch Vorsehen von zusätzlichen Werkstoffen auszubilden.

Entlang der Längsachse verändern sich die Anteile zwischen longitudinalen und querschwingenden Schwingungskomponenten. Die Dämpfungsstruktur ist bevorzugt im Bereich eines Amplitudenmaximums der Querschwingungen bzw. eines maximalen Anteils der Querschwingungen angeordnet. Durch die Konversionsstruktur werden die eingeleiteten Longitudinalschwingungen entlang der Längsachse des Sonotrodenkörpers in Querschwingungen umgewandelt. Dabei verändern sich die Anteile der longitudinalen Schwingungen und der Querschwingungen entlang der Längsachse.

Die Konversionsstruktur erstreckt sich typischerweise über eine Länge in Längsrichtung der Ultraschallkomponente, die etwa 10 % bis 30 %, bevorzugt etwa 15 % bis 25 % der Länge des Sonotrodenkörpers erstreckt. Es hat sich gezeigt, dass mit einer derartigen Länge eine besonders optimale Konversion von longitudinalen Schwingungen in Querschwingungen erzielbar ist.

Ausserdem ist die Konversionsstruktur bevorzugt in Längsrichtung gesehen aussermittig zwischen der Dämpfungsstruktur und der ersten Stirnfläche angeordnet, und zwar näher zur Stirnfläche hin. Dadurch wird zwischen der Konversionsstruktur und der Dämpfungsstruktur ein Zwischenbereich gebildet. In diesem Zwischenbereich ändert sich in Längsrichtung betrachtet laufend die Verteilung zwischen dem Anteil an longitudinalen Schwingungen und Querschwingungen. Die Länge dieses Zwischenbereichs wird so gewählt, dass im Bereich der Dämpfungsstruktur der Anteil an Querschwingungen maximiert ist.

In einer bevorzugten Ausführungsform ist ausserdem auf der Schalleinleitungsseite eine Vertiefung in der ersten Stirnfläche angeordnet. Die Vertiefung ist mit einer Koppelfläche zum Verbinden des Sonotrodenkörpers mit einer Schwingfläche eines Ultraschallkonverters versehen. Auf diese Art und Weise kann eine optimierte Einkopplung der longitudinalen Schwingungen erzielt werden. Insbesondere kann eine Anordnung wie in der pendenten Anmeldung EP 18210827.4 verwendet werden, deren Inhalt durch Querverweis zum Gegenstand der vorliegenden Anmeldung gemacht wird.

Gemäss einem weiteren bevorzugten Ausführungsbeispiel kann an der Aussenfläche des Sonotrodenkörpers benachbart zu einem Bereich mit maximaler Amplitude der Querschwingungen, insbesondere im Bereich der Dämpfungsstruktur, eine Schwingungsmasse vorgesehen sein. Typischerweise erfolgt die Einleitung der Querschwingungen in das Werkstück auf der zweiten Stellfläche der Bearbeitungsseite. Es ist aber auch denkbar, zusätzlich oder alternativ Schwingungen im Bereich einer solchen Schwingungsmasse in Werkstücke einzuleiten.

Der Sonotrodenkörper kann typischerweise einstückig ausgebildet sein und beispielsweise aus Stahl, Titan, Aluminium oder aus einer Keramik bestehen. Es ist aber auch denkbar, mehrteilige Sonotrodenkörper vorzusehen. In diesem Zusammenhang ist es insbesondere denkbar, auch Sonotrodenkörper aus mehreren Teilen aus unterschiedlichen Materialien zu verwenden. Insbesondere ist es denkbar, zwischen der Bearbeitungsseite und der Schalleinleitungsseite im Bereich der Dämpfungsstruktur ein zusätzliches, dämpfendes Material vorzusehen.

Die Erfindung betrifft ausserdem eine Vorrichtung zum Bearbeiten von Werkstücken mittels Ultraschalles. Die Vorrichtung weist wenigstens eine wie vorstehend beschriebene Ultraschallkomponente auf. Ausserdem weist die Vorrichtung einen Konverter zum Betreiben der Ultraschallkomponente und insbesondere zum Einleiten von longitudinalen Schwingungen in die Schalleinleitungsseite der Ultraschallkomponente auf. Die Vorrichtung weist ausserdem eine Aufnahme für ein zu bearbeitendes Werkstück auf. Ausserdem verfügt die Vorrichtung über eine Betätigungsvorrichtung zum Bewegen der Ultraschallkomponente gegen die Aufnahme hin. Damit kann in an sich bekannter Art und Weise ein Werkstück zwischen eine Bearbeitungsfläche der Ultraschallkomponente und der Aufnahme eingeklemmt und mit Ultraschall beaufschlagt werden.

Die Erfindung betrifft schliesslich ein Verfahren zum Bearbeiten von Werkstücken mittels Ultraschalles. Insbesondere wird dabei eine Ultraschallkomponente wie vorstehend beschrieben verwendet. In einem ersten Schritt werden longitudinale Schwingungen an einer Schalleinleitungsseite eines Sonotrodenkörpers der Ultraschallkomponente in die Ultraschallkomponente eingeleitet. Die eingeleiteten longitudinalen Schwingungen werden mittels einer Konversionsstruktur in Querschwingungen mit einer Schwingungskomponente in einer Ebene senkrecht zur Schwingungsrichtung der longitudinalen Schwingungen transformiert.

Diese Querschwingungen werden in ein Werkstück auf einer Bearbeitungsseite der Sonotrode eingeleitet oder in eine weitere Ultraschallkomponente auf der Bearbeitungsseite eines Boosters eingeleitet. Erfindungsgemäss werden die longitudinalen Schwingungen zwischen der Konversionsstruktur und der Bearbeitungsseite mittels einer insbesondere longitudinalen Dämpfungsstruktur reduziert.

Die Erfindung wird nachfolgend anhand der Zeichnungen und in Ausführungsbeispielen näher erläutert. Es zeigen:
- Figur 1: eine schematische Darstellung einer erfindungsgemässen Vorrichtung;
- Figur 2: eine Seitenansicht einer erfindungsgemässen Sonotrode;
- Figur 3: eine perspektivische Darstellung einer ersten alternativen Form einer Sonotrode;
- Figur 4: eine Seitenansicht einer zweiten alternativen Ausführungsform einer Sonotrode;
- Figur 5: eine Seitenansicht einer dritten alternativen Ausführungsform einer Sonotrode;
- Figur 6: eine perspektivische, schematische Darstellung einer vierten alternativen Ausführungsform einer erfindungsgemässen Sonotrode und
- Figur 7: eine Seitenansicht einer fünften alternativen Ausführungsform einer erfindungsgemässen Sonotrode.

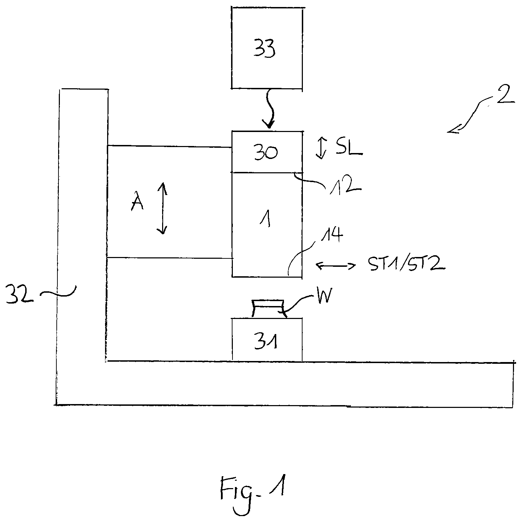

Figur 1 zeigt schematisch eine Vorrichtung 2 zum Bearbeiten von Werkstücken W. Als Werkstücke W sind hier exemplarisch zwei Teile gezeigt, welche miteinander verschweisst werden sollen. Die Vorrichtung 2 weist eine Aufnahme 31 zur Aufnahme der Werkstücke W auf.

Eine Sonotrode 1 ist mittels eines Konverters 30 zur Ultraschallschwingungen anregbar. Der Konverter 30 wird durch einen Ultraschallgenerator 33 zu Longitudinalschwingungen SL in einer longitudinalen Richtung angeregt. In der Sonotrode 1 werden die an einer ersten Stirnfläche 12 eingekoppelten Longitudinalschwingungen SL in Querschwingungen konvertiert, sodass an einer zweiten Stirnfläche 14 Querschwingungen, beispielsweise torsionale Schwingungen ST1 oder transversale Schwingungen ST2, entstehen und in das Werkstück W eingeleitet werden können. Der Stack aus Konverter 30 und Sonotrode 1 ist mittels eines Antriebs in Achsrichtung A bewegbar, sodass die Sonotrode 1 mit der Stirnfläche 14 in Richtung des Werkstücks W bewegt werden kann. Zum Bewegen des Stacks in Achsrichtung A ist typischerweise ein pneumatischer Antrieb 32 vorgesehen. Denkbar sind aber auch Servopressen in an sich bekannter Art und Weise.

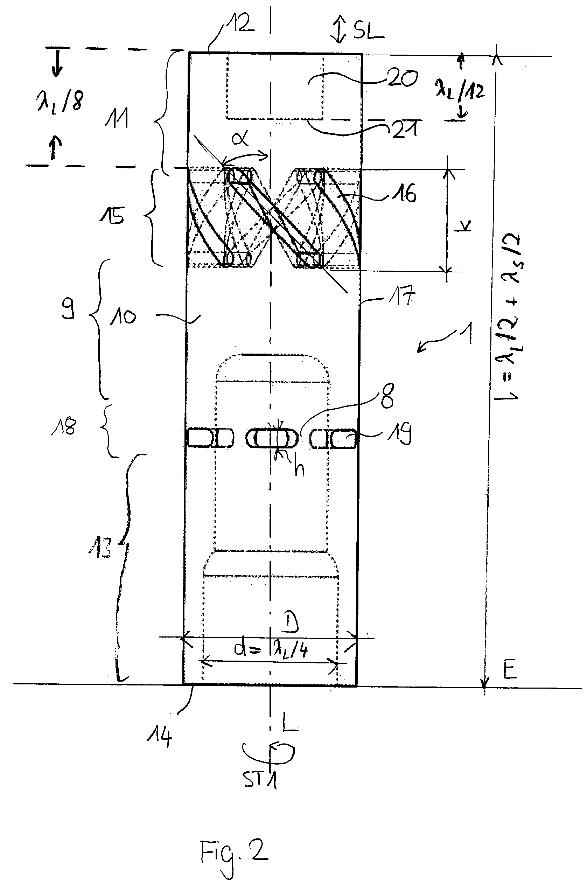

Figur 2 zeigt eine Seitenansicht einer ersten Ausführungsform einer erfindungsgemässen Sonotrode 1. Die Sonotrode 1 weist einen im Wesentlichen zylindrischen Sonotrodenkörper 10 auf. Der Sonotrodenkörper 10 weist eine Schalleinleitungsseite 11 mit einer ersten Stirnfläche 12 und eine Bearbeitungsseite 13 mit einer zweiten Stirnfläche 14 auf. An der ersten Stirnfläche 12 sind longitudinale Schwingungen SL in Längsrichtung L der Sonotrode 1 einleitbar. Zu diesem Zweck weist der Sonotrodenkörper 10 an der ersten Stirnseite 12 eine sacklochartige Vertiefung 20 mit einer Grundfläche 21 auf. Mit der Grundfläche 21 ist ein Konverter verbindbar, sodass longitudinale Schwingungen SL in die Sonotrode 1 einkoppelbar sind. In Längsrichtung L anschliessend an die Schalleinleitungsseite 11 befindet sich eine Konversionsstruktur 15 in Form von Konversionsschlitzen 16. Die Konversionsschlitze 16 sind mit einer Neigung bezogen auf die Längsachse L auf einer Oberfläche 17 des Sonotrodenkörpers 10 angeordnet. Die Konversionsschlitze 16 führen dazu, dass aus den longitudinalen Schwingungen SL, welche auf der Schalleinleitungsseite 11 in die Sonotrode 10 eingeleitet werden, Schwingungen mit einer torsionalen Komponente erzeugt werden. In einem Zwischenbereich 9, welcher in Längsrichtung L an die Konversionsstruktur 15 folgt, bestehen Schwingungen mit sowohl longitudinalen als auch torsionalen Schwingungskomponenten. Der Anteil der einzelnen Schwingungskomponenten variiert in Längsrichtung L.

An den Zwischenbereich 9 schliesst sich ein Dämpfungsbereich 18 an. Der Dämpfungsbereich 18 ist durch Dämpfungsschlitze 19 gebildet, welche sich in Umfangsrichtung entlang der Oberfläche 17 des Sonotrodenkörpers 10 erstrecken. Die Schlitze 19 sind regelmässig über den Umfang des Sonotrodenkörpers 10 angeordnet und durch Stege 8 voneinander getrennt. In Längsrichtung L gesehen nach der Dämpfungsstruktur 18 befindet sich die Bearbeitungsseite 13. Die Stege 8 verbinden den Zwischenbereich 9 mit der Bearbeitungsseite 13. Über die Stege 8 werden torsionale Schwingungskomponenten auf die Bearbeitungsseite 13 übertragen, sodass diese fast nur noch ausschliesslich mit torsionalen Schwingungen ST1 schwingt. Auf der Bearbeitungsseite 13 liegen kaum Schwingungen in Längsrichtung L vor. Die torsionalen Schwingungen ST1 verlaufen in einer Ebene E senkrecht zur Längsachse L.

Der Sonotrodenkörper 10 weist eine totale Länge l auf,. die in Abhängigkeit von Material und Frequenz bestimmt wird. Im hier dargestellten Ausführungsbeispiel ist die Länge l gleich der Summe einer halben longitudinalen Wellenlänge und einer halben torsionalen Wellenlänge.

Die Konversionsschlitze 16 weisen in Längsrichtung L von eine Länge k auf, die bevorzugt im Bereich von D/6 bis D liegt, wobei D der Aussendurchmesser des Sonotrodenkörpers 10 ist. Im hier dargestellten Ausführungsbeispiel ist die Länge k ca. 13 % der Gesamtlänge l des Sonotrodenkörpers 10. Die Konversionsschlitze 16 verlaufen unter einem Winkel α von ungefähr 45° bezogen auf die Längsachse L.

Der Zwischenbereich 9 weist in der Ausführungsform gemäss Figur 2 typischerweise eine Länge auf, die kleiner ist als eine halbe longitudinale Wellenlänge. Die Bearbeitungsseite 13 weist eine Länge von typischerweise einer halben torsionalen Wellenlänge auf. Die Konversionsschlitze 16 beginnen typischerweise in einem Abstand von ungefähr einem Achtel der longitudinalen Wellenlänge von der ersten Stirnfläche 12 und/oder in einem Abstand von maximal einem Zwölftel der longitudinalen Wellenlänge von der Koppelfläche 21 zum Einleiten von Longitudinalschwingungen SL.

In Längsrichtung L betrachtet weisen die Dämpfungsschlitze 19 typischerweise eine Höhe h von 1 mm bis 10 mm auf. Ähnliche Dimensionen gelten auch für die nachfolgenden Ausführungsbeispiele.

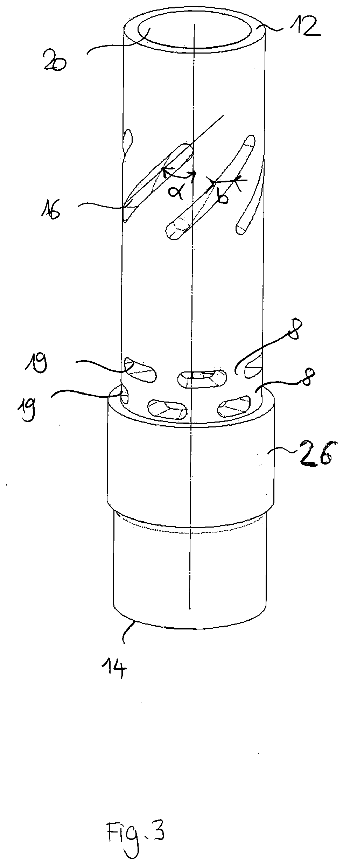

Figur 3 zeigt eine erste alternative Sonotrode 1 in einer perspektivischen Darstellung. Gleiche Bezugszeichen bezeichnen gleiche Bauteile. Die Konversionsschlitze 16 befinden sich auf Schraubenlinien S, welche unter einem Winkel α bezogen auf die Längsachse L verlaufen. Die Schraubenlinie S hat hier eine veränderliche Steigung, sodass der Winkel α nicht in jedem Punkt der Konversionsschlitze 16 gleich ist. Im Ausführungsbeispiel gemäss den Figuren 2 und 3 sind jeweils sechs Konversionsschlitze 16 angeordnet. In Umfangsrichtung gesehen weisen die Konversionsschlitze 16 eine Breite b von 1 mm bis 10 mm auf.

Der Sonotrodenkörper 10 gemäss Figuren 2 und 3 ist als teilweiser Hohlkörper ausgebildet. Der Sonotrodenkörper 10 weist einen kreisringförmigen Querschnitt auf. Der Querschnitt weist einen Aussendurchmesser D von typischerweise einem Viertel der longitudinalen Wellenlänge und einen insbesondere vom bearbeiteten Werkstück abhängigen Innendurchmesser d von etwa einem Achtel der longitudinalen Wellenlänge auf. Die Konversionsschlitze 16 sind als Vertiefungen ausgebildet und die Dämpfungsschlitze 19 erstrecken sich durch die ganze Wand des hohlzylindrischen Sonotrodenkörpers 10.

Im Ausführungsbeispiel gemäss Figuren 2 und 3 sind im Umfangsrichtung gesehen sechs Dämpfungsschlitze 19 angeordnet. Die Länge der Dämpfungsschlitze 19 und der Stege 8 in Umfangsrichtung ist in diesem Ausführungsbeispiel etwa gleich gross. Die Stege 8 und die Dämpfungsschlitze 19 erstrecken sich jeweils zusammen über einen Winkelbereich von ca. 60°. Die Mittelpunkte der Dämpfungsschlitze 19 gemäss Figur 2 fluchten in Richtung der Längsachse L mit den Mittelpunkten der Konversionsschlitze 16; in alternativen Ausführungsbeispielen können die genannten Mittelpunkte natürlich auch bezüglich der Längsachse L gegeneinander versetzt sein. In Figur 3 sind zwei Reihen von Dämpfungsschlitzen vorgesehen, die bezogen aufeinander in Umfangsrichtung betrachtet um etwa 30° versetzt sind. Im Unterschied zu Figur 2 weist die Sonotrode 1 nach Figur 3 ein zusätzliches umlaufendes Transformationsstück 26 auf.

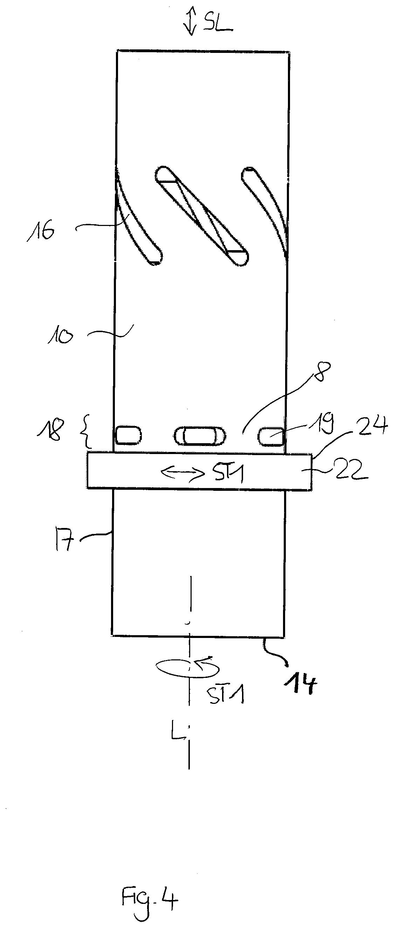

Figur 4 zeigt eine Sonotrode 1 einer zweiten alternativen Ausführungsform in einer Seitenansicht. Die Sonotrode 1 gemäss Figur 4 hat einen Sonotrodenkörper 10, der im Wesentlichen dem Sonotrodenkörper 10 gemäss Figur 2 entspricht. Insbesondere sind auch hier Konversionsschlitze 16 vorgesehen, welche longitudinale Schwingungen SL in torsionale Schwingungen ST1 konvertieren. Die torsionalen Schwingungen ST1 weisen eine tangentiale Schwingungsrichtung um die Längsachse L auf. Die Verteilung der longitudinalen und torsionalen Komponenten hängt von der Anordnung und Grösse der Konversionsschlitze 16 ab. Die Struktur 18 ist in Längsrichtung L gesehen an einer Stelle angeordnet, an der der Anteil an torsionalen Schwingungen ST1 möglichst gross wird. Mittels FEM-Berechnungen kann der Schwingungsverlauf auf dem Sonotrodenkörper 10 ermittelt werden. Es können danach gezielt Dämpfungsschlitze 19 und eine Schwingungsmasse 22 an derjenigen Stelle angebracht werden, an der die torsionale Komponente am grössten sein soll.

Im Unterschied zu Figur 2 ist bei der Sonotrode 1 gemäss Figur 4 zusätzlich eine Schwingungsmasse 22 vorgesehen. Die Schwingungsmasse 22 verläuft in Umfangsrichtung gesehen um den Sonotrodenkörper 10 herum. Die Schwingungsmasse 22 ist benachbart zur Dämpfungsstruktur 18 angeordnet. Aufgrund der Schwingungsmasse 22 bestehen an von der Oberfläche 17 radial vorstehenden Oberflächenbereichen 24 der Schwingungsmasse 22 zusätzliche torsionale Schwingungen. Entsprechend können Schwingungen in Werkstücke eingeleitet werden, indem Werkstücke in Kontakt mit der zweiten Stirnfläche 14 oder in Kontakt mit der Oberfläche 24 der Schwingungsmasse 22 gebracht werden. Dazu kann die Oberfläche 24 mit geeigneten Konturen oder Vorsprüngen versehen werden.

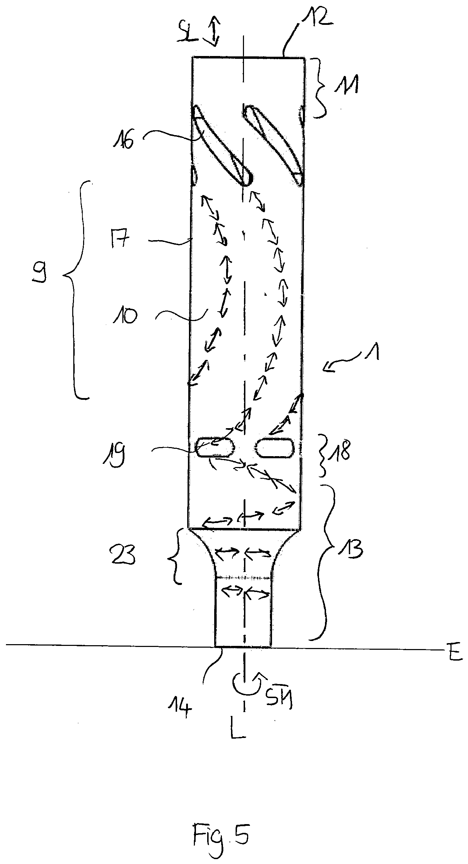

Figur 5 zeigt eine weitere Ausführungsform einer erfindungsgemässen Sonotrode 1. Der Sonotrodenkörper 10 in der Ausführungsform gemäss Figur 5 ist im Gegensatz zu den Figuren 2 bis 4 als voller Körper ausgebildet. Die Konversionsschlitze 16 und die Dämpfungsschlitze 19 sind entsprechend nicht als durchgehende Schlitze sondern nur als Vertiefungen auf der Oberfläche 17 des Sonotrodenkörpers 10 ausgebildet. Im Übrigen bezeichnen gleiche Bezugszeichen in Figur 5 die gleichen Komponenten wie in den Figuren 2, 3 und 4. Im Unterschied zu den Ausführungsformen gemäss Figuren 2, 3 und 4 weist der Sonotrodenkörper 10 ausserdem auf der Bearbeitungsseite 13 eine Verjüngung 23 auf. Im Bereich der Verjüngung 23 reduziert sich der Aussendurchmesser des Sonotrodenkörpers 10 entlang der Längsachse.

In Figur 5 ist ausserdem schematisch der Verlauf der Schwingungen innerhalb des Sonotrodenkörpers 10 gezeigt.

Auf der Schalleinleitungsseite 11 werden Longitudinalschwingungen SL eingeleitet, welche in Längsrichtung L verlaufen. Aufgrund der Konversionsschlitze 16 ergibt sich im Bereich der Konversionsstruktur 15 eine Schwingung mit zunehmender torsionaler Komponente.

Im Zwischenbereich 9 nimmt der torsionale Anteil an den Schwingungen zuerst zu und dann wieder ab, sodass die torsionalen Schwingungen ungefähr in der Mitte des Zwischenbereichs 9 minimal sind. Dort liegt ein torsionaler Schwingungsknoten vor. Gegen die Dämpfungsstruktur 18 hin nimmt die torsionale Komponente der Schwingungen dann wieder zu. Ungefähr im Bereich der Dämpfungsstruktur 18 ist die torsionale Schwingung maximal.

Aufgrund der Dämpfungsschlitze 19 werden auf die Bearbeitungsseite 13 kaum mehr longitudinale Schwingungsanteile übertragen, sodass im Bereich der Bearbeitungsseite 13 hauptsächlich torsionale Schwingungen ST1 mit einer Schwingungsrichtung tangential um die Längsachse L und in einer Ebene E senkrecht zur Längsachse L bestehen.

Mit den erfindungsgemässen Sonotroden, insbesondere den Sonotroden gemäss den Figuren 2 bis 5, lassen sich Verhältnisse zwischen torsionalen und linearen Amplituden von mehr als 1:1, bevorzugt mehr als 2:1, beispielsweise 2:1, 3:1 oder 4:1, erzielen.

Dank der Einkopplung der longitudinalen Schwingungen LS im Bereich der Vertiefung 20 gemäss Figur 2 und 3 lässt sich ausserdem eine Amplitudenübersetzung von 1 zu 4,3 erzielen.

Je nach verwendeter Ultraschallfrequenz können selbstverständlich die Sonotroden skaliert werden.

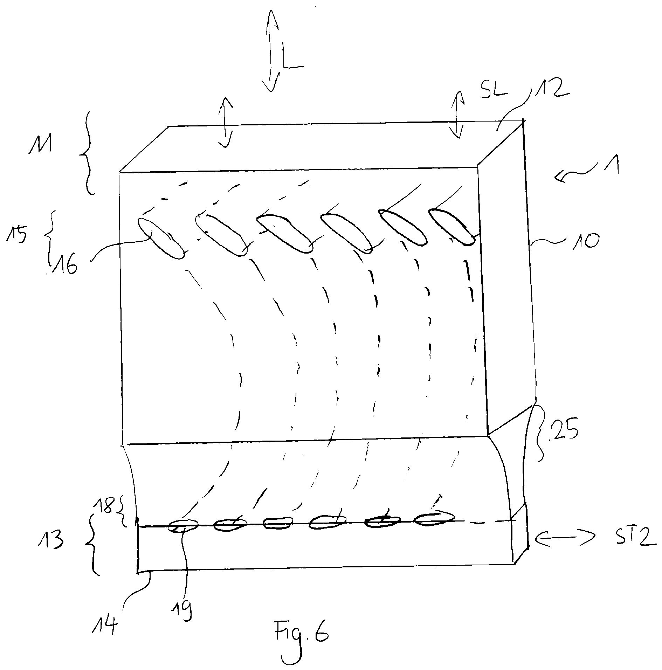

In Figur 6 ist eine weitere alternative Ausführungsform einer Sonotrode 1 gezeigt. Hier ist der Sonotrodenkörper 10 rechteckig ausgebildet. Longitudinale Schwingungen SL werden an der ersten Stirnseite 12 eingeleitet. Durch Konversionsschlitze 16 im Bereich einer Konversionsstruktur 15 werden die longitudinalen Schwingungen SL ähnlich wie im Zusammenhang mit den Figuren 2 bis 5 beschrieben in Schwingungen mit einer quer verlaufenden Schwingungskomponente konvertiert. Aufgrund der rechteckigen Ausführung des Sonotrodenkörpers 10 werden hier transversale Schwingungen ST2 erzeugt, welche im Wesentlichen linear in einer Schwingungsrichtung senkrecht zur Schwingungsrichtung L der longitudinalen Schwingung SL verlaufen. Der Sonotrodenkörper 10 weist gegen die zweite Stirnfläche 14 hin eine Verjüngung 25 auf. In einem Dämpfungsbereich 18 sind auch hier Dämpfungsschlitze 19 vorgesehen, welche zwischen der Bearbeitungsseite 13 und der Konversionsstruktur 15 angeordnet sind.

Die Sonotrode gemäss Figur 6 ist typischerweise als "blade" eingesetzt. Mittels solcher Sonotroden 1 lassen sich Materialbahnen, beispielsweise Folienbahnen, miteinander verschweissen und trennen. Typischerweise können solche Sonotroden bei der Herstellung von Verpackungen eingesetzt werden.

Die Sonotroden aus Figuren 2 bis 5 werden hingegen typischerweise zum Verschweissen von Kunststoffteilen verwendet, wenn die Teile mit der zweiten Stirnfläche 14 in Kontakt gebracht werden. Es ist aber auch denkbar, Metallteile, beispielsweise Litzen, zu verschweissen, die in Kontakt mit einer Oberfläche 24 der Schwingungsmasse 22 der Ausführungsform gemäss Figur 4 in Kontakt gelangen.

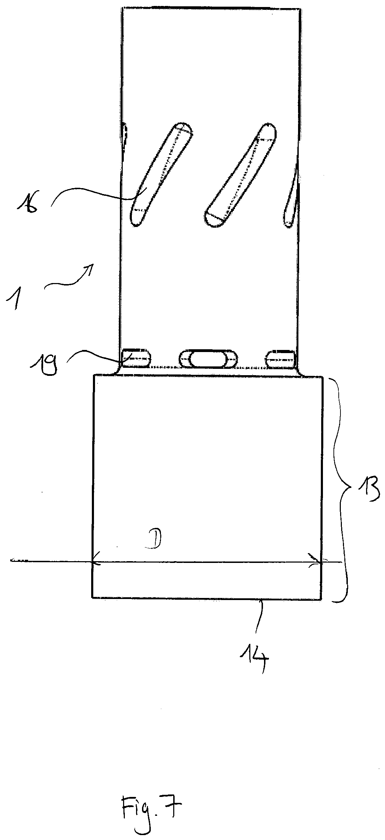

Figur 7 zeigt eine weitere Ausführungsform einer Sonotrode 1. Hier ist der Aussendurchmesser D der Bearbeitungsseite vergrössert.

## Patentansprüche

1. Ultraschallkomponente, insbesondere Sonotrode (1) oder Booster, zum Bearbeiten von Werkstücken (W) mit Ultraschallschwingungen,
mit einem Sonotrodenkörper (10) mit einer Längsachse (L), wobei der Sonotrodenkörper eine Schalleinleitungsseite (11) mit einer ersten Stirnfläche (12) und eine Bearbeitungsseite (13) mit einer zweiten Stirnfläche (14) aufweist,
wobei auf der Schalleinleitungsseite (11) longitudinale Schwingungen (SL) in einer Richtung parallel zur Längsachse (L) in den Sonotrodenkörper (10) einleitbar sind,
wobei zwischen der Schalleinleitungsseite (11) und der Bearbeitungsseite (13) eine Konversionsstruktur (15) angeordnet ist, mittels deren aus den longitudinalen Schwingungen (SL) Querschwingungen (ST1; ST2) mit einer Schwingungskomponente in einer Ebene (E) senkrecht zur Längsachse (L) erzeugbar sind,
**dadurch gekennzeichnet, dass** zwischen der Konversionsstruktur (15) und der Bearbeitungsseite (13) eine Dämpfungsstruktur (18) vorgesehen ist, welche die longitudinalen Schwingungen (SL) auf der Bearbeitungsseite (13) reduziert.

2. Ultraschallkomponente (1) nach Anspruch 1, wobei der Sonotrodenkörper (10) ganz oder teilweise als Hohlkörper ausgebildet ist.

3. Ultraschallkomponente (1) nach Anspruch 1 oder 2, wobei der Sonotrodenkörper (10) rotationssymmetrisch, insbesondere mit kreisförmigem Querschnitt, ausgebildet ist und die Querschwingungen torsionale Schwingungen (ST) sind.

4. Ultraschallkomponente (1) nach Anspruch 3, wobei der Sonotrodenkörper (10) einen Aussendurchmesser (D) von weniger als einem Viertel der longitudinalen Wellenlänge aufweist.

5. Ultraschallkomponente (1) nach einem der Ansprüche 1 bis 4, wobei die Konversionsstruktur (15) in Form von Materialaussparungen (16) an der Aussenfläche (17) des Sonotrodenkörpers (10) ausgebildet ist, welche sich entlang einer Schraubenlinie (S) erstrecken, insbesondere in Form von Konversionsschlitzen (16), wobei die Schraubenlinie (S) bevorzugt einen Winkel von 45° zur Längsachse (L) aufweist.

6. Ultraschallkomponente (1) nach einem der Ansprüche 1 bis 5, wobei die Dämpfungsstruktur (18) durch eine Materialschwächung im Sonotrodenkörper (10) gebildet ist.

7. Ultraschallkomponente (1) nach Anspruch 6, wobei die Materialschwächung der Dämpfungsstruktur (18) in Form von Dämpfungsschlitzen (19) ausgebildet ist, welche sich in einer Richtung senkrecht zur Längsachse (L) erstrecken.

8. Ultraschallkomponente (1) einem der Ansprüche 1 bis 7, wobei die Dämpfungsstruktur (18) benachbart zu einem Bereich eines maximalen Anteils an Querschwingungen (ST1; ST2) angeordnet ist.

9. Ultraschallkomponente (1) nach einem der Ansprüche 1 bis 8, wobei sich die Konversionsstruktur (15) über eine Länge (k) von ca. 10 % bis 30 %, bevorzugt etwa 1 0% bis 20 % der Länge (1) des Sonotrodenkörpers (10) erstreckt.

10. Ultraschallkomponente (1) nach einem der Ansprüche 1 bis 9, wobei die Konversionsstruktur (15) in Längsrichtung (L) gesehen aussermittig zwischen der Dämpfungsstruktur (18) und der ersten Stirnfläche (12) angeordnet ist.

11. Ultraschallkomponente (1) nach einem der Ansprüche 1 bis 10, wobei auf der Schalleinleitungsseite (11) eine Vertiefung (20) mit einer Koppelfläche (21) zum Verbinden mit einer Schwingfläche eines Ultraschallkonverters (30) vorgesehen ist.

12. Ultraschallkomponente (1) nach einem der Ansprüche 1 bis 11, wobei an der Aussenfläche (17) des Sonotrodenkörpers (10) insbesondere benachbart zu einem Bereich mit maximaler Amplitude der Querschwingungen, insbesondere zum Bereich der Dämpfungsstruktur (18), eine Schwingungsmasse (22) vorgesehen ist.

13. Ultraschallkomponente (1) nach einem der Ansprüche 1 bis 12, wobei der Sonotrodenkörper (10) einstückig ausgebildet ist und insbesondere aus Stahl, Titan oder einer Keramik ausgebildet ist.

14. Vorrichtung (2) zum Bearbeiten von Werkstücken (W) mittels Ultraschalles, mit wenigstens einer Ultraschallkomponente (1) nach einem der Ansprüche 1 bis 13, mit einem Konverter (30) zum Einleiten von longitudinalen Schwingungen (SL) in die Schalleinleitungsseite (11), mit einer Aufnahme (31) für ein zu bearbeitendes Werkstück (W) und mit einer Betätigungsvorrichtung (32) zu Bewegen der Ultraschallkomponente (1) gegen die Aufnahme (31) hin.

15. Verfahren zum Bearbeiten von Werkstücken (W) mittels Ultraschalles, insbesondere mit einer Ultraschallkomponente (1) nach einem der Ansprüche 1 bis 13, mit den Schritten
- Einleiten von longitudinalen Schwingungen (SL) an einer Schalleinleitungsseite (11) eines Sonotrodenkörpers (10) der Ultraschallkomponente (1),
- Transformieren der longitudinalen Schwingungen (SL) in Querschwingungen (ST1; ST2) mit einer Schwingungskomponente in einer Ebene (E) senkrecht zur Schwingungsrichtung der longitudinalen Schwingungen (SL) mittels einer Konversionsstruktur (15),
- Einleiten der Querschwingungen (SL1; SL2) in ein Werkstück (W) auf einer Bearbeitungsseite (13) der Sonotrode (1) oder in eine weitere Ultraschallkomponente auf der Bearbeitungsseite eines Boosters,
**dadurch gekennzeichnet, dass** die longitudinalen Schwingungen (SL) zwischen der Konversionsstruktur (15) und der Bearbeitungsseite (13) mittels einer insbesondere longitudinalen Dämpfungsstruktur (18) reduziert werden.
